(19) Europäisches Patentamt
European Patent Office
Office européen des brevets

(11) Numéro de publication: 0 211 759 B1

# (12) FASCICULE DE BREVET EUROPEEN

(45) Date de publication de fascicule du brevet: 17.04.91

(51) Int. Cl.5: C07D 513/04, A61K 31/505, //(C07D513/04,277:00,239:00)

(21) Numéro de dépôt: 86401707.4

(22) Date de dépôt: 31.07.86

(54) **Nouveaux dérivés du pyrimido /2,1-b/ benzothiazole et leurs sels, procédé de préparation, application à titre de médicaments et compositions les renfermant.**

(30) Priorité: 31.07.85 GB 8519261

(43) Date de publication de la demande: 25.02.87 Bulletin 87/09

(45) Mention de la délivrance du brevet: 17.04.91 Bulletin 91/16

(84) Etats contractants désignés: AT BE CH DE FR IT LI LU NL SE

(56) Documents cités:
EP-A- 0 005 154
EP-A- 0 153 230
US-A- 3 919 201

CHEMICAL ABSTRACTS, vol. 91, 1979, page 638, résumé no. 193220g, Columbus, Ohio, US; KANG-CHIEN LIU et al.: "Synthesis and bioactivities of some 2-oxonaphto[1,2-d]thiazolo(3,2-a)pyrimidine derivatives", & ARCH. PHARM. (WEINHEIM, GER.) 1979, 312(7), 619-22

(73) Titulaire: ROUSSEL-UCLAF
35, boulevard des Invalides
F-75007 Paris(FR)

(72) Inventeur: Rowlands, David Alun
Wyke House
Crudwell Malmesbury Wiltshire SN16 9ET(GB)
Inventeur: Matharu, Saroop Shingh
1 Hopkins Orchard
Cricklade Wiltshire SN6 6EB(GB)
Inventeur: Hairsine, Peter Wilfred
39 Hermin Street
Stratton St Margaret Swindon Wiltshire(GB)

(74) Mandataire: Vieillefosse, Jean-Claude et al
Département des Brevets ROUSSEL UCLAF
B.P no 9
F-93230 Romainville(FR)

EP 0 211 759 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a pour objet de nouveaux dérivés du pyrimido /2,1-b/ benzothiazole, ainsi que leurs sels d'addition avec les acides, le procédé de préparation et l'application à titre de médicament de ces nouveaux produits.

Le brevet américain US A.3919.201 décrit des produits présentant à la fois des substituants différents sur le noyau tricyclique en position 8 et des propriétés pharmacologiques différentes (antifungiques, antiamibiens et antiinflammatoires).

La demande de brevet européen EP A.0153.230 (non publiée à la date de priorité de la présente demande) décrit des dérivés du pyrimido /2-1-b/ benzothiazole et leurs sels, qui different de ceux revendiqués dans la présente demande par la longueur du radical alcoxy $R_1$ porté par la fonction :

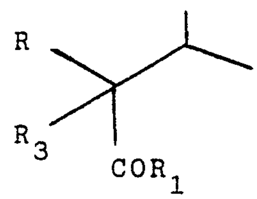

L'invention a pour objet de nouveaux dérivés du pyrimido /2,1-b/ benzothiazole ainsi que leurs sels d'addition avec les acides, caractérisés en ce qu'ils répondent à la formule générale (I) :

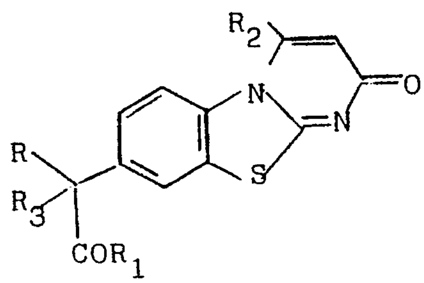

(I)

dans laquelle R et $R_3$, identiques ou différents, représentent un atome d'hydrogène, un radical alcoyle, linéaire ou ramifié, renfermant de 1 à 6 atomes de carbone ou R et $R_3$ forment avec l'atome de carbone auquel ils sont liés, un radical cycloalcoyle renfermant de 3 à 6 atomes de carbone, $R_1$ représente un radical alcoxy, linéaire, ramifié ou cyclisé, renfermant de 7 à 12 atomes de carbone, R2 représente un atome d'hydrogène, un radical alcoyle, linéaire ou ramifié, renfermant de 1 à 6 atomes de carbone, un radical aryle renfermant de 6 à 12 atomes de carbone, un radical aryle renfermant de 6 à 12 atomes de carbone substitué par un ou plusieurs radicaux alcoyles ou alcoxy renfermant de 1 à 6 atomes de carbone, par un ou plusieurs atomes d'halogène ou par un ou plusieurs radicaux nitro ou $R_2$ représente un radical 2-thiényle, un radical alcoxycarbonyle renfermant de 2 à 7 atomes de carbone ou un radical cycloalcoyle renfermant de 3 à 6 atomes de carbone.

Dans la formule générale (I) et dans ce qui suit, le terme radical alcoyle renfermant de 1 à 6 atomes de carbone désigne par exemple un radical méthyle, éthyle, propyle, isopropyle, butyle, isobutyle, pentyle ou hexyle ;

le terme radical alcoxy linéaire, ramifié ou cyclisé, renfermant de 7 à 12 atomes de carbone désigne, par exemple, un radical heptyloxy, octyloxy, nonyloxy, décyloxy, 1-méthylcyclohexyloxy ou adamantyloxy ;

le terme radical cycloalcoyle renfermant de 3 à 6 atomes de carbone désigne de préférence le radical cyclohexyle ;

le terme radical aralcoyle renfermant de 7 à 13 atomes de carbone désigne de préférence un radical benzyle ;

le terme radical aryle renfermant de 6 à 12 atomes de carbone désigne par exemple un radical phényle ou naphtyle ;

le terme radical alcoxycarbonyle renfermant de 2 à 7 atomes de carbone désigne par exemple un radical méthoxy, éthoxy ou propoxy-carbonyle ;

le terme atome d'halogène désigne par exemple un atome de fluor, de chlore ou de brome;

le terme radical alcoxy renfermant de 1 à 6 atomes de carbone désigne, par exemple, un radical méthoxy,

éthoxy, propoxy, isopropoxy, butoxy, pentoxy ou hexyloxy ;

Les produits de formule (I) présentent un caractère basique et peuvent par conséquent former des sels d'addition avec les acides tels que, par exemple, les sels formés avec les acides chlorhydrique, bromhydrique, iodhydrique, nitrique, sulfurique, phosphorique, acétique, formique, benzoïque, maléique, fumarique, succinique, tartrique, citrique, oxalique, glyoxylique, aspartique, alcane sulfoniques, tels que l'acide méthane sulfonique et aryl sulfoniques, tels que l'acide benzène sulfonique.

Parmi les produits objet de l'invention, on peut citer notamment les dérivés répondant à la formule (I) ci-dessus, ainsi que leurs sels d'addition avec les acides, caractérisés en ce que dans ladite formule (I), R et $R_3$ représentent un atome d'hydrogène ou un radical méthyle ou éthyle, $R_1$ et $R_2$ ont la signification déjà indiquée.

Parmi les produits objet de l'invention, on retient plus particulièrement les dérivés répondant à la formule (I) ci-dessus, ainsi que leurs sels d'addition avec les acides, caractérisés en ce que dans la dite formule (I), R et $R_3$ représentent un atome d'hydrogène ou un radical méthyle ou éthyle, $R_1$ a la signification déjà indiquée, $R_2$ représente un atome d'hydrogène, un radical alcoyle renfermant de 1 à 6 atomes de carbone, un radical phényle, un radical phényle substitué par un ou plusieurs radicaux méthyle par un ou plusieurs atomes d'halogène ou par un ou plusieurs radicaux nitro ou méthoxy ou $R_2$ représente un radical méthoxy carbonyle.

Parmi ceux-ci, on retient plus particulièrement les dérivés répondant à la formule (I) ci-dessus ainsi que leurs sels d'addition avec les acides, caractérisés en ce que dans ladite formule (I), R et $R_3$ représentent un atome d'hydrogène ou un radical méthyle ou éthyle, $R_2$ représente un radical phényle et $R_1$ représente un radical 1-méthyl cyclohexyloxy ou un radical 1-adamantyloxy.

Parmi ces derniers, on peut citer tout particulièrement les dérivés du pyrimido /2,1-b/ benzothiazole dont les noms suivent :

- l'α-méthyl 2-oxo 4-phényl 2H-pyrimido /2,1-b/ benzothiazole 8-acétate de 1-méthylcyclohexyle,
- l'α-méthyl 2-oxo 4-phényl 2H-pyrimido /2,1-b/ benzothiazole-8-acétate de 1-adamantyle,

ainsi que leurs sels d'addition avec les acides.

L'invention a également pour objet un procédé de préparation des nouveaux dérivés du pyrimido /2,1-b/ benzothiazole tels que d´finis ci-dessus, ainsi que leurs sels d'addition avec les acides, caractérisé en ce que l'on fait réagir un produit de formule (II) :

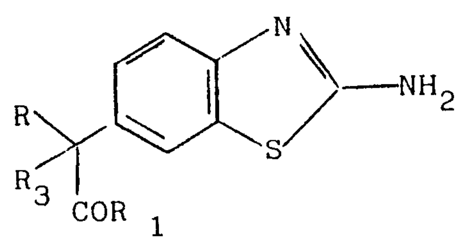

(II)

dans laquelle R, $R_1$ et $R_3$ ont la signification déjà indiquée, avec un produit de formule (III) :

$$R_2-C\equiv C-CO_2R_4 \qquad (III)$$

dans laquelle $R_2$ a la signification déjà indiquée et $R_4$ représente un radical alcoyle renfermant de 1 à 3 atomes de carbone, pour obtenir le produit de formule (I) correspondant, que soit l'on isole et si désiré salifie, soit l'on fait réagir ce dernier avec un alcool de formule (IV) :

$$H-R'_1 \qquad (IV)$$

dans laquelle $R'_1$ représente un radical alcoxy linéaire, ramifié ou cyclisé renfermant de 7 à 12 atomes de carbone pour obtenir un composé de formule (I) correspondant dans laquelle $R_1$ a la signification de $R'_1$ que l'on isole et si désiré salifie.

Dans des conditions préférentielles de mise en oeuvre de l'invention, le procédé de préparation ci-dessus décrit est caractérisé en ce que la réaction du produit de formule (II) avec le propiolate de formule (III) peut être effectuée, au reflux du mélange réactionnel, au sein d'un alcanol et en présence d'un

catalyseur. L'alcanol peut-être L'éthanol, le catalyseur peut-être le palladium.
La réaction est effectuée de préférence par chauffage à une température de 100 à 180¤C, en l'absence de solvant.

L'invention a également pour objet un procédé de préparation des nouveaux dérivés du pyrimido /2,1-b/ benzothiazole tels que définis ci-dessus, ainsi que leurs sels d'addition avec les acides, caractérisé en ce que l'on fait réagir un produit de formule (V) :

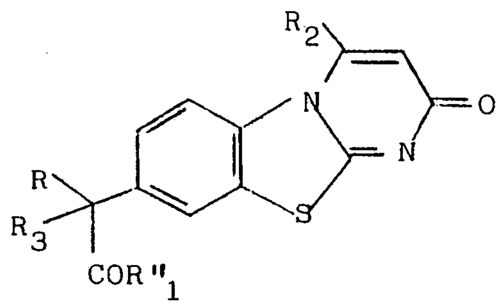

(V)

dans laquelle R, $R_2$ et $R_3$ ont la signification déjà indiquée et $R''_1$ représente un radical hydroxy ou alcoxy renfermant de 1 à 6 atomes de carbone, avec un alcool de formule (VI) :

$$H-R_1 \qquad (VI)$$

dans laquelle $R_1$ a la signification déjà indiquée, pour obtenir un produit de formule (I) correspondant que l'on isole et si désiré salifie.

Les sels peuvent être préparés en faisant réagir en proportions sensiblement stoechiométriques, un acide minéral ou organique avec un produit de formule (I).

Les produits objet de la présente invention possèdent de très intéressantes propriétés pharmacologiques ; ils sont doués notamment de remarquables propriétés antiallergiques.

Ces propriétés sont illust rées plus loin dans la partie expérimentale.

Ces propriétés justifient l'utilisation des nouveaux dérivés du pyrimido /2,1-b/ benzothiazole, objet de la présente l'invention, ainsi que de leurs sels pharmaceutiquement acceptables, à titre de médicaments.

La présente demande a ainsi également pour objet l'application à titre de médicaments des nouveaux dérivés du pyrimido /2,1-b/ benzothiazole tels que définis par la formule (I), ainsi que de leurs sels d'addition avec les acides pharmaceutiquement acceptables.

Parmi les médicaments objet de l'invention, on retient de préférence les médicaments caractérisés en ce qu'ils sont constitués par les dérivés répondant à la formule (I) dans laquelle R et $R_3$ représentent un atome d'hydrogène ou un radical méthyle ou éthyle, $R_1$ et $R_2$ ont la signification déjà indiquée, ainsi que par leurs sels d'addition avec les acides pharmaceutiquement acceptables.

Parmi ceux-ci, on retient notamment les médicaments caractérisés en ce qu'ils sont constitués par les dérivés répondant à la formule (I) dans laquelle R et $R_3$ représentent un atome d'hydrogène ou un radical méthyle ou éthyle, $R_1$ a la signification déjà indiquée, $R_2$ représente un atome d'hydrogène, un radical alcoyle renfermant de 1 à 6 atomes de carbone, un radical phényle, un radical phényle substitué par un ou plusieurs radicaux méthyle, par un ou plusieurs atomes d'halogène, ou par un ou plusieurs radicaux nitro ou méthoxy ou $R_2$ représente un radical méthoxy carbonyle, ainsi que par leurs sels d'addition avec les acides pharmaceutiquement acceptables.

Parmi ceux-ci, on retient plus particulièrement les médicaments caractérisés en ce qu'ils sont constitués par les dérivés répondant à la formule (I) dans laquelle R et $R_3$ représentent un atome d'hydrogène, un radical méthyle ou éthyle, $R_2$ représente un radical phényle et $R_1$ représente un radical 1-méthylcyclohexyloxy ou un radical 1-adamantyloxy ainsi que par leurs sels d'addition avec les acides pharmaceutiquement acceptables.

Parmi ces derniers, on retient tout particulièrement les produits dont les noms suivent :

- l'α-méthyl 2-oxo 4-phényl 2H-pyrimido /2,1-b/ benzothiazole 8-acétate de l-méthylcyclohexyle,
- l'α-méthyl 2-oxo 4-phényl 2H-pyrimido /2,1-b/ benzothiazole-8-acétate de 1-adamantyle,

ainsi que leurs sels d'addition avec les acides pharmaceutiquement acceptables.

Ces médicaments trouvent, par exemple, leur emploi dans le traitement de l'asthme allergique et des

bronchites asthmatiformes d'origine allergique.

La dose usuelle, variable selon le produit utilisé, le sujet traité et l'affection en cause peut être, par exemple, de 1 mg à 1000 mg par jour, par voie orale chez l'homme.

L'invention a également pour objet les compositions pharmaceutiques qui renferment au moins un dérivé précité ou l'un de ses sels d'addition avec les acides pharmaceutiquement acceptables, à titre de principe actif.

A titre de médicaments, les dérivés répondant à la formule (I) et leurs sels d'addition avec les acides pharmaceutiquement acceptables peuvent être incorporés dans des compositions pharmaceutiques destinées à la voie digestive, parentérale ou locale.

Ces compositions pharmaceutiques peuvent être par exemple, solides ou liquides et se présenter sous les formes pharmaceutiques couramment utilisées en médecine humaine, comme, par exemple, les comprimés, simples ou dragéifiés, les gélules, les granulés, les suppositoires, les sirops, les aérosols, les crèmes, les pommades, les préparations injectables ; elles sont préparées selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale o u végétale, les dérivés paraffiniques, les glycols, les divers agents mouillants, dispersants ou émulsifiants, les conservateurs.

Certains produits de formule (II) sont décrits dans la littérature et notamment par S. N. Sawhney et al dans Ind. J. Chem . 16 B , 605 (1978), ainsi que dans la demande allemande 2.015.158, dans le brevet US 3.656.958 et dans la demande européenne 17543 A.

Toutefois, certains composés de formule (II) sont nouveaux.

Les produits de formule (II) dans laquelle R et $R_3$ représentent un atome hydrogène, lorsqu'ils ne sont pas connus, peuvent être préparés selon le schéma suivant :

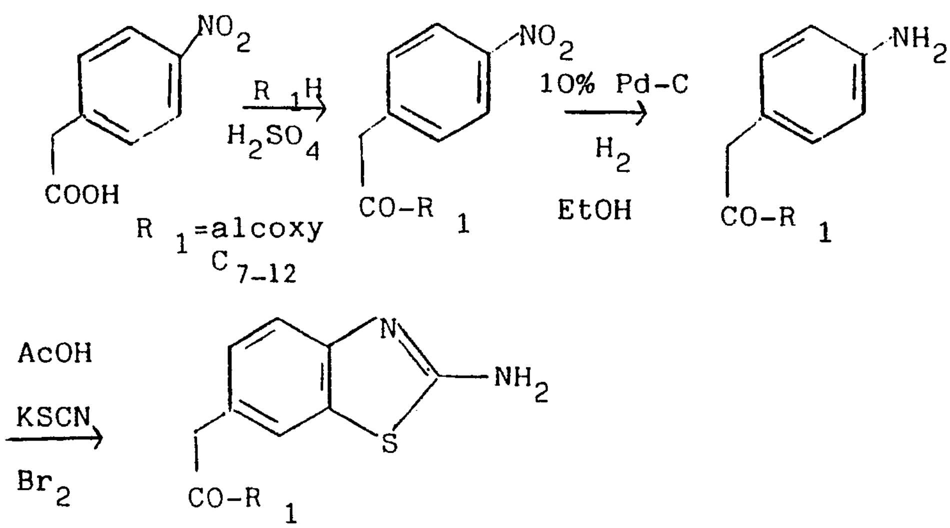


Les produits de formule (II) peuvent être également préparés au départ d'α-phényl aldéhydes selon le schéma suivant :

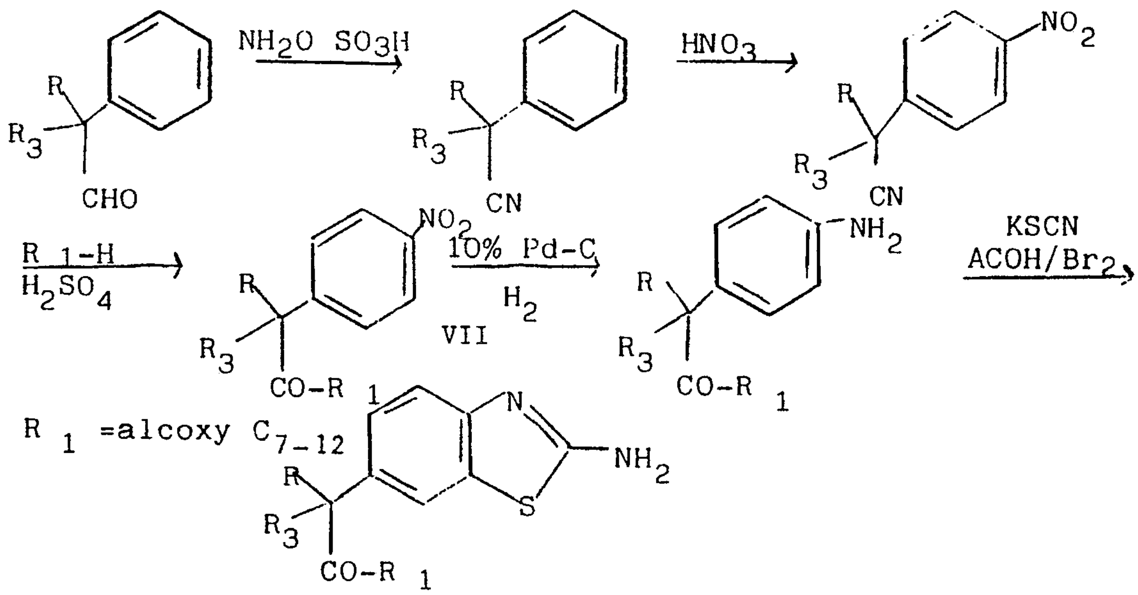


Lorsque l'on veut préparer selon le procédé ci-dessus des produits dans lesquels R et $R_3$ représentent un radical alcoyle, il est préférable de préparer l'intermédiaire de formule (VII) :

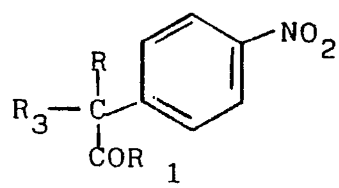

(VII)

par alcoylation d'un produit de formule (VIII) :

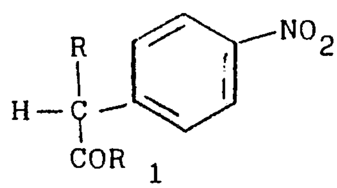

(VIII)

dans laquelle R représente un radical alcoyle et $R_1$ a la signification déjà indiquée, en utilisant, par exemple un iodure d'alcoyle $R_3I$ et une base telle que le diisopropylamidure de lithium ou le N-isopropylcyclohexyl amidure de lithium, au sein d'un solvant tel que le tétrahydrofuranne.

La préparation des produits de formule (VIII) est décrite plus loin.

Les produits de formule (II) dans laquelle

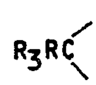

représente un radical

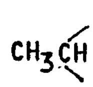

peuvent, selon une variante, également être préparés selon le schéma suivant :

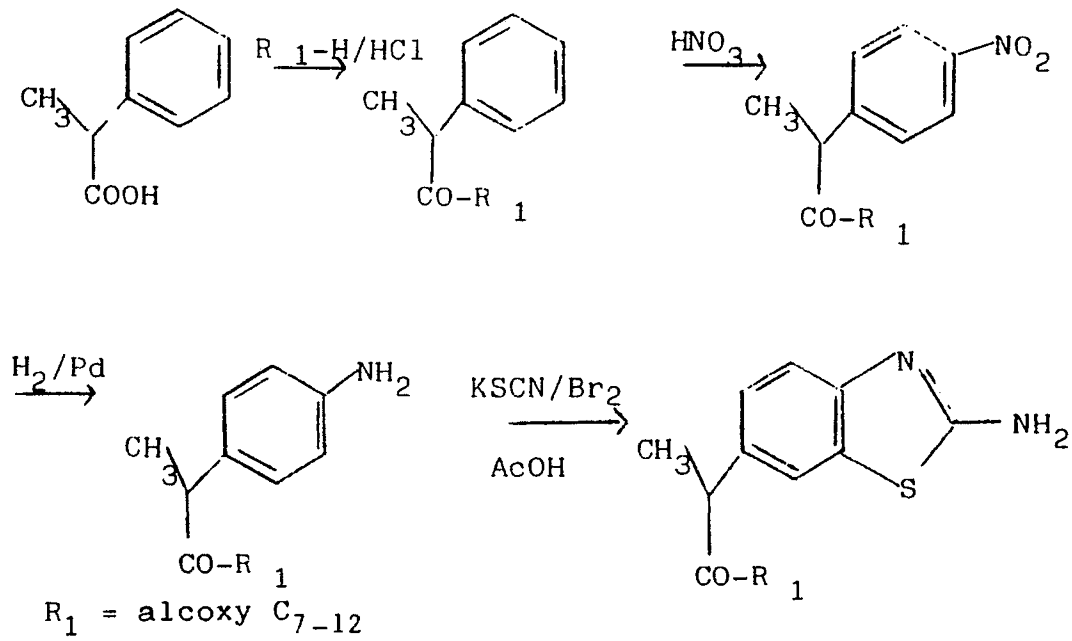


Les produits de formule (II) dans laquelle $R_3$ (ou R) représente un atome d'hydrogène, peuvent aussi être préparés au départ de 1-chloro 4-nitrobenzène selon le schéma suivant :

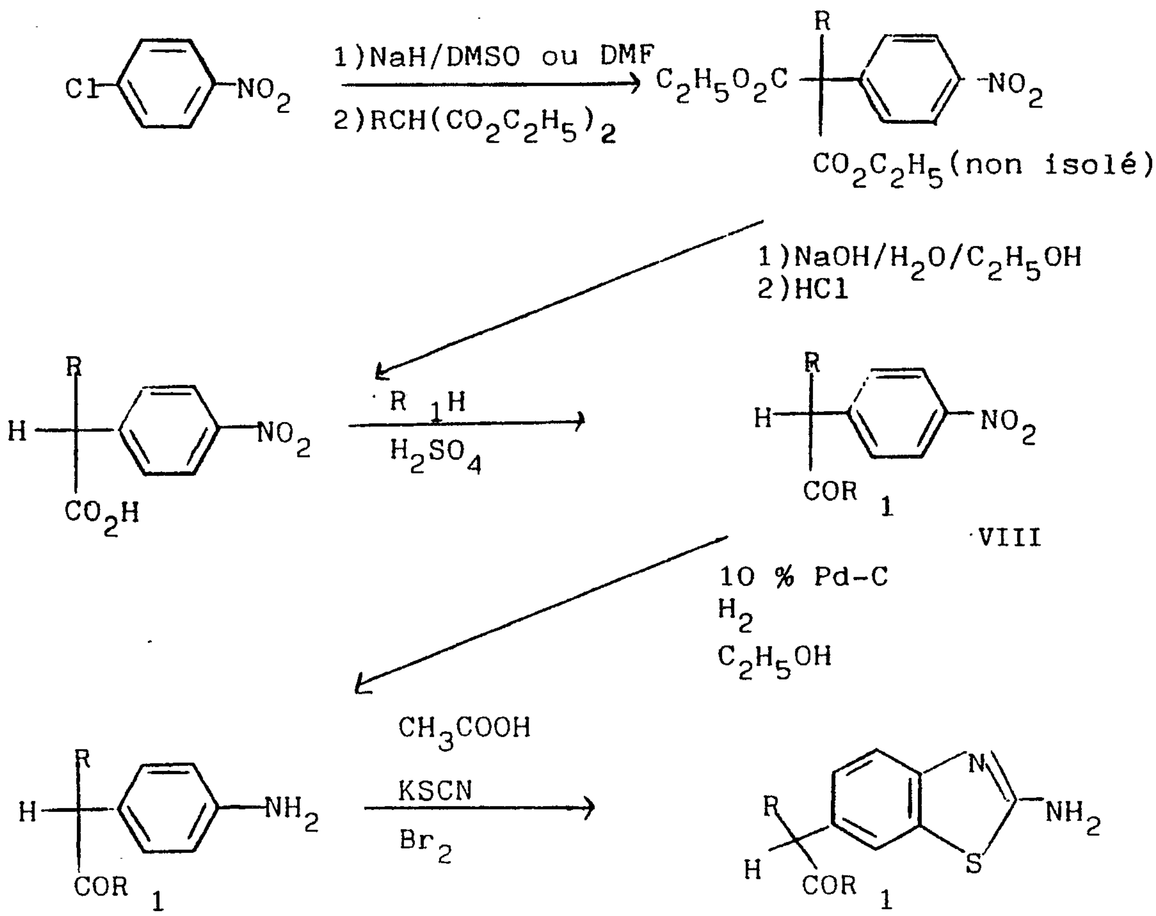


Dans le schéma qui précède R est un atome d'hydrogène ou un radical alcoyle $C_{1-6}$ de préférence méthyle ou éthyle et $R_1$ a la signification indiquée précédemment.

Les premiers stades sont inspirés du procédé décrit par K.HINO et al.j.Med.Chem. 26 222-226 (1983).

les produits de formule :

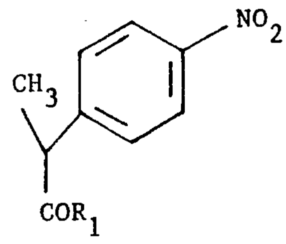

dans laquelle $R_1$ a la signification déjà indiquée peuvent être préparés au départ de l'acide 2-(p-nitrophényl) propionique selon le schéma suivant :

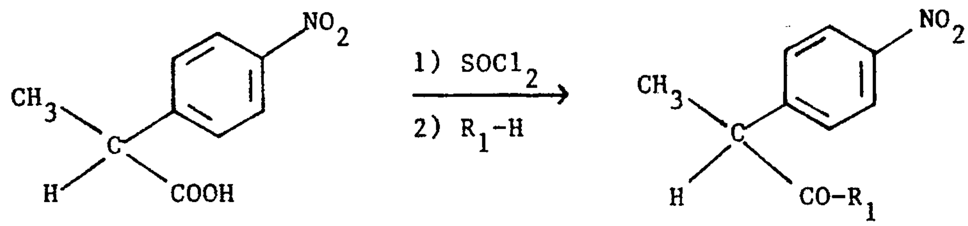


Les composés de formule (V) dans laquelle R, $R_2$ et $R_3$ ont la définition déjà indiquée et $R''_1$ représente un radical hydroxy ou un radical alcoxy renfermant de 1 à 6 atomes de carbone peuvent être préparés selon le procédé décrit dans la demande de brevet européen n¤ 0 153 230.

Il va être donné maintenant à titre non limitatif des exemples de mise en oeuvre de l'invention.

**Exemple 1 : α-méthyl 2-oxo 4-phényl 2H-pyrimido /2,1-b/ benzothiazole 8-acétate de 1-méthylcyclohexyle.**

**Stade A :** 2-(p-nitrophényl) propionate de 1-méthylcyclohexyle.

On chauffe à 80¤C pendant 5 heures sous agitation 97,5 g d'acide 2-(p-nitrophényl) propionique (préparé comme dans J. Med. Chem., 26 , 222-226 (1983) dans 40 cm3 de chlorure de thionyle et 500 cm3 de toluène. On refroidit et élimine le solvant sous pression réduite. On dissout le résidu huileux dans 400 cm3 de toluène et ajoute 100 g de 1-méthylcyclohexanol. On chauffe à 80¤C le mélange pendant 1 heure puis abandonne 16 heures à température ambiante. On lave la solution à l'aide d'une solution aqueuse de bicarbonate de soude à 5%, puis à l'eau, sèche et concentre à sec. On chromatographie le résidu sur silice (éluant : chloroforme) et récupère 34,95 g de produit attendu.

Spectre IR

2940 $cm^{-1}$ - 1725 $cm^{-1}$ (ester)

1520 $cm^{-1}$ - 1345 $cm^{-1}$ -1150 $cm^{-1}$.

**Stade B :** 2-(p-aminophényl) propionate de 1-méthylcyclohexyle.

On hydrogène sous pression ambiante 34,9 g de produit obtenu au stade A dans 250 cm3 d'éthanol en présence de palladium à 10% sur charbon actif. On filtre le catalyseur, évapore le solvant, reprend le résidu à l'éther de pétrole et obtient 21,02 g de produit attendu. F = 85-87¤C.

Spectre IR

3640 $cm^{-1}$ (-$NH_2$) - 3370 $cm^{-1}$ (-$NH_2$) - 2940 $cm^{-1}$ - 1710 $cm^{-1}$ - 1215 $cm^{-1}$.

**Stade C :** 2-amino α-méthylbenzothiazole 6-acétate de 1-méthylcyclohexyle.

On chauffe à 50¤C sous agitation 20,9 g de produit obtenu au stade B et 31 g de thiocyanate de potassium dans 150 cm3 d'acide acétique et ajoute en 30 minutes 25,6 g de brome en solution dans 15 cm3 d'acide acétique. On maintient sous agitation à 50¤C pendant 30 minutes, refroidit et verse dans 1 litre de mélange acétate d'éthyle-eau (2-3). On ajuste le mélange réactionnel à pH 5-6 à l'aide de bicarbonate de soude, filtre, lave la solution organique à l'eau, la sèche et la concentre à sec. On reprend le résidu par de l'éther de pétrole et récupère 18,62 g de produit attendu. F = 165-167¤C.

Spectre IR

3410 $cm^{-1}$ - 2930 $cm^{-1}$ - 1710 $cm^{-1}$(ester) - 1550 $cm^{-1}$ - 1215 $cm^{-1}$.

**Stade D :** α-méthyl 2-oxo 4-phényl 2H-phényl 2H-pyrimido /2,1-b/ benzothiazole 8-acétate de 1-méthylcyclohexyle.

On chauffe à 160¤C pendant 30 minutes, 3,2 g de produit obtenu au stade C et 3,5 g de phénylpropiolate d'éthyle en ajoutant peu à peu 3,5 g de phénylpropiolate d'éthyle supplémentaire. On refroidit à +60¤C puis ajoute avec précaution 30 cm3 d'éther. On refroidit et obtient 1,82 g de produit attendu cristallisé. F = 168-170¤C.

Spectre IR

2930 $cm^{-1}$ - 1720 $cm^{-1}$ (ester) - 1610 $cm^{-1}$ - 1510 $cm^{-1}$ - 1145 $cm^{-1}$.

**Analyse :**

| | | | | |
|---|---|---|---|---|
| Calculé : | C% 69,93 | H% 5,87 | N% 6,27 | S% 7,18 |
| Trouvé : | 69,87 | 5,90 | 6,23 | 7,23. |

**Exemple 2 : α-méthyl 2-oxo 4-phényl 2H-pyrimido /2,1-b/ benzothiazole 8-acétate de 1-adamantyle.**

**Stade A** : 2-(p-nitrophényl propionate de 1-adamantyle.

En utilisant une méthode analogue à celle décrite à l'exemple 1 stade A, mais au départ de 14,6 g de 2-(-nitrophényl) propionate et en remplaçant le 1-méthylcyclohexanol par du 1-adamantanol, on obtient 22,74 g de produit attendu.

Spectre IR

2920 $cm^{-1}$ - 1725 $cm^{-1}$ (ester) - 1520 $cm^{-1}$ - 1345 $cm^{-1}$ - 1055 $cm^{-1}$.

**Stade B** : 2-(p-aminophényl) propionate de 1-adamantyle.

On opère selon une méthode analogue à celle décrite au stade B de l'exemple 1 en utilisant 21,4 g de produit préparé comme au stade précédent at obtient 19,42 g de produit attendu.

Spectre IR

3450 $cm^{-1}$ ($NH_2$) - 3370 $cm^{-1}$ ($NH_2$) - 2910 $cm^{-1}$ - 1720 $cm^{-1}$ (ester) 1055 $cm^{-1}$.

**Stade C** : 2-amino α-méthylbenzothiazole 6-acétate de 1-adamantyle.

On opère comme au stade C de l'exemple 1 au départ de 19,1 g de produit obtenu au stade précédent et obtient 18,97 g de produit attendu. F = 151-153¤C.

Spectre IR

3400 $cm^{-1}$ - 2910 $cm^{-1}$ - 1710 $cm^{-1}$ (ester) - 1540 $cm^{-1}$ - 1210 $cm^{-1}$.

**Stade D** : α-méthyl 2-oxo 4-phényl 2H-pyrimido /2,1-b/ benzothiazole 8-acétate de 1-adamantyle.

On opère comme à l'exemple 1 stade D en utilisant 8,9 g de produit préparé au stade précédent et en chauffant 1 heure à 160¤C. Après recristallisaion dans un mélange éther de pétrole-acétat e d'éthyle, on obtient 4,73 g de produit attendu. F = 188-190¤C.

Spectre IR

2910 $cm^{-1}$ - 1720 $cm^{-1}$ (ester) - 1640 $cm^{-1}$ - 1510 $cm^{-1}$ - 1390 $cm^{-1}$.

**Analyse :**

| | | | | |
|---|---|---|---|---|
| Calculé : | C% 71,88 | H% 5,82 | N% 5,78 | S% 6,62 |
| Trouvé : | 71,71 | 5,87 | 5,75 | 6,57. |

**Exemple 3** :

On a préparé des comprimés répondant à la formulation suivante :

Produit de l'exemple 1 .......................... 15 mg

Excipient q.s.pour un comprimé terminé à........... 100 mg.

(Détail de l'excipient : lactose, amidon, talc, stéarate de magnésium).

**Exemple 4** :

On a préparé un aérosol délivrant par dose :

Produit de l'exemple 2 .......................... 2 mg
Emulsifiant.......................................... 0,15 mg
Propulseur........................................... 50 mg.

Exemple 5 :

On a préparé des comprimés répondant à la formulation suivante :

Produit de l'exemple 2 .......................... 15 mg
Excipient q.s. pour un comprimé terminé à.......... 100 mg.
(Détail de l'excipient : lactose, amidon, talc, stéarate de magnésium).

ETUDE PHARMACOLOGIQUE.

Augmentation de la pression de perfusion dans les poumons induite par un antigène.

On utilise pour ce test des cochons d'Inde mâles Dunkin Harley (Porcellus) pesant 450 à 700 g (à raison de 4 animaux par dose de produit à tester).

Les animaux sont sensibilisés par exposition biheddomadaire à un aérosol contenant de l'ovalbumine (1 % poids/volume).

Après avoir été anesthésiés au moyen d'une injection intra-péritonéale de diazépam (2,5 mg/kg) et d'une injection intra-musculaire d'hypnorm (1 ml/kg), les animaux sont exsanguinés par section des carotides.

On ouvre la cage thoracique, prélève les poumons, les partage en 2 (au niveau du sternum) et les relie à un système de perfusion après avoir introduit une canule dans une bronche du lobe principal.

Les poumons sont perfusés avec du liquide de Krebs oxygéné (95 % d'oxygène - 5 % de gaz carbonique) à 37¤C.

On injecte une solution de l'ovalbumine à raison de 5 g pour 0,1 ml à proximité de chaque poumon. On enregistre l'augmentation de la pression de la perfusion due à l'antigène.

60 minutes plus tard, on administre 15µg d'ovalbumine. Les produits à tester sont ajoutés au liquide de Krebs 30 minutes avant la 2ème administration d'antigène.

Lors de chaque série de traitements, on effectue des mesures de contrôle sur des animaux témoins ne recevant aucun produit test (n = 10).

La réponse au second antigène administré, est exprimée en pourcentage du premier.

Pour chaque administration de produit à tester, on utilise au moins 4 poumons (de 4 animaux différents, on calcule le pourcentage d'inhibition de la bronchoconstriction induite par l'antigène. ($CI_{50}$ en µM).

Les résultats sont donnés dans le Tableau 1 ci-dessous.

| Produit de l'exemple | $CI_{50}$ µM |
|---|---|
| 1 | 1-10 |
| 2 | $\leq 1$ |

## Revendications

1. Nouveaux dérivés du pyrimido /2,1-b/ benzothiazole ainsi que leurs sels d'addition avec les acides, caractérisés en ce qu'ils répondent à la formule générale (I) :

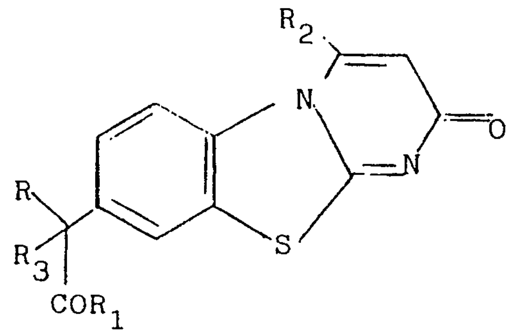

(I)

dans laquelle R et $R_3$ indentiques ou différents, représentent un atome d'hydrogène, un radical alcoyle, linéaire ou ramifié, renfermant de 1 à 6 atomes de carbone ou R et $R_3$ forment avec l'atome de carbone auquel ils sont liés, un radical cycloalcoyle renfermant de 3 à 6 atomes de carbone, $R_1$ représente un radical alcoxy, linéaire, ramifié ou cylisé renfermant de 7 à 12 atomes de carbone, $R_2$ représente un atom d'hydrogène, un radical alcoyle, linéaire ou ramifié, renfermant de 1 à 6 atomes de carbone, un radical aryle renfermant de 6 à 12 atomes de carbone, un radical aryle renfermant de 6 à 12 atomes de carbone substitué par un ou plusieurs radicaux alcoyles ou alcoxy renfermant de 1 à 6 atomes de carbone, par un ou plusieurs atomes d'halogène ou par un ou plusieurs radicaux nitro, ou $R_2$ représente un radical 2-thiényle, un radical alcoxy carbonyle renfermant de 2 à 7 atomes de carbone ou un radical cycloalcoyle renfermant 3 à 6 atomes de carbone.

2. Dérivés répondant à la formule générale (I) de la revendication 1, ainsi que leurs sels d'addition avec les acides, caractérisés en ce que dans ladite formule (I), R et $R_3$ représentent un atome d'hydrogène ou un radical méthyle ou éthyle, $R_1$ et $R_2$ ont la signification déjà indiquée.

3. Dérivés répondant à la formule générale (I) de la revendication 1, ainsi que leurs sels d'addition evec les acides, caratérisés en ce que dans ladite formule (I), R et $R_3$ représentent un atome d'hydrogène ou un radical méthyle ou éthyle, $R_1$ a la signification déjà indiquée, $R_2$représente un atome d'hydrogène, un radical alcoyle renfermant de 1 à 6 atomes de carbone, un radical phényle, un radical phényle substitué par un ou plusieurs radicaux méthyle, par un ou plusieurs atomes d'halogène, ou par un ou plusieurs radicaux nitro ou méthoxy ou $R_2$ représente un radical méthoxy carbonyle.

4. Dérivés répondant à la formule (I) de la revendication 1, ainsi que leurs sels, caractérisés en ce que dans ladite formule (I), R et $R_3$ représentent un atome d'hydrogène ou un radical méthyle ou éthyle, $R_2$ représente un radical phényle et $R_1$ représente un radical 1-méthylcyclohexyloxy ou un radical 1-adamantyloxy.

5. L'un quelconque des produits de formule (I) telle que définie à la revendication 1, dont les noms suivent :

   - l'α-méthyl-2-oxo-4-phényl 2H-pyrimido /2,1-b/ benzothiazole 8-acétate de 1-méthylcyclohexyle,
   - l'α-méthyl 2-oxo 4-phényl 2H-pyrimido /2,1-b/ benzothiazole 8-acétate de 1-adamantyle. ainsi que leurs sels d'addition avec les acides.

6. Procédé de préparation des nouveaux dérivés du pyrimido /2,1-b/ benzothiazole ainsi que de leurs sels d'addition avec les acides, caractérisé en ce que l'on fait réagir un produit de formule (II) :

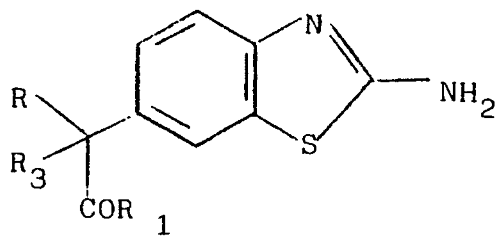

(II)

dans laquelle R, $R_1$ et $R_3$ ont la signification indiquée à la revendication 1, avec un produit de formule (III) :

$$R_2 - C \equiv C - CO_2R_4 \quad (III)$$

dans laquelle $R_2$ a la signification déjà indiquée et $R_4$ représente un radical alcoyle renfermant de 1 à 3 atomes de carbone, pour obtenir le produit de formule (I) correspondant, que soit l'on isole et si désiré salifie, soit l'on transestérifie par action d'un alcool de formule (IV) :

$$H-R'_1 \quad (IV)$$

dans laquelle $R'_1$ représente un radical alcoxy linéaire, ramifié ou cyclisé renfermant de 7 à 12 atomes de carbone pour obtenir un compose de formule (I) correspondant dans laquelle $R_1$ a la signification de $R'_1$ que l'on isole et si désiré, salifie.

7. Procédé de préparation des nouveaux dérivés du pyrimido /2,1-b/ benzothiazole ainsi que de leurs sels d'addition avec les acides, caractérisé en ce que l'on fait réagir un produit de formule (V) :

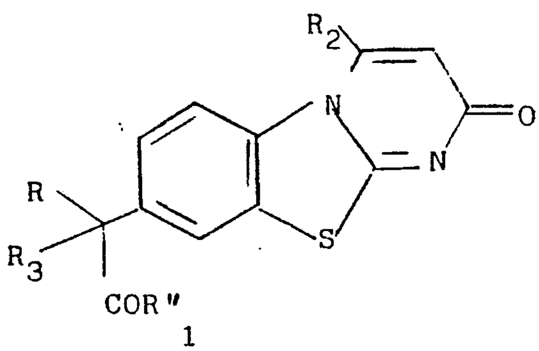

(V)

dans laquelle R, $R_2$ et $R_3$ ont la signification déjà indiquée et $R''_1$ représente un radical hydroxy ou alcoxy renfermant de 1 à 6 atomes de carbone, avec un alcool de formule (VI) :

$$H-R_1 \quad (VI)$$

dans laquelle $R_1$ a la signification déjà indiquée, pour obtenir un produit de formule (I) correspondant que l'on isole et si désiré salifie.

8. Procédé de préparation des nouveaux dérivés du pyrimido /2,1-b/ benzothiazole tel que défini à la revendication 6, caractérisé en ce que

la réaction du produit de formule (II) avec le produit de formule (III) est effectuée au reflux du mélange réactionnel, au sein d'un alcanol et en présence d'un catalyseur.

9. Procédé selon la revendication 8, caractérisé en ce que la réaction du produit (II) avec le produit de formule (III) est effectuée au sein de l'éthanol en présence de palladium.

10. Procédé de préparation des nouveaux dérivés du pyrimido /2,1-b/ benzothiazole tel que défini à la revendication 6, caractérisé en ce que la réaction du produit de formule (II) avec le produit de formule (III) est effectuée, à une température de 100 à 180 °C, en l'absence de solvant.

11. Médicaments, caractérisés en ce qu'ils sont constitués par les nouveaux dérivés du pyrimido /2,1-b/ benzothiazole tels que définis par la formule (I) de la revendication 1 ainsi que par leurs sels d'addition avec les acides pharmaceutiquement acceptables.

12. Médicaments, caractérisés en ce qu'ils sont constitués par les nouveaux dérivés tels que définis à l'une quelconque des revendications 2 à 5, ainsi que par leurs sels d'addition avec les acides pharmaceutiquement acceptables.

13. Compositions pharmaceutiques, caractérisées en ce qu'elles renferment à titre de principe actif, l'un au moins des médicaments tels que définis à l'une quelconque des revendications 11 ou 12.

Revendications pour l'Etat contractant suivant : AT

1. Procédé de préparation de nouveaux dérivés du pyrimido /2,1-b/ benzothiazole ainsi que de leurs sels d'addition avec les acides, les acides, répondant à la formule générale (I) :

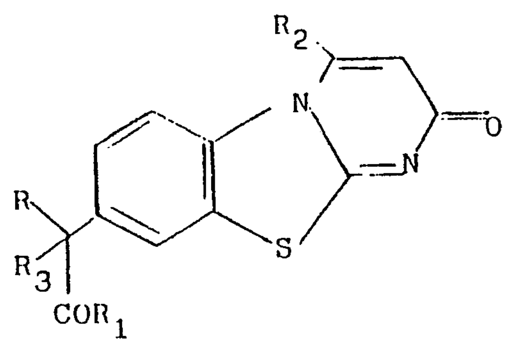

(I)

dans laquelle R et $R_3$, identiques ou différents, différents, représentent un atome d'hydrogène, un radical alcoyle, linéaire ou ramifié, renfermant de 1 à 6 atomes de carbone ou R et $R_3$ forment avec l'atome de carbone auquel ils sont liés, un radical cycloalcoyle renfermant de 3 à 6 atomes de carbone, $R_1$ représente un radical alcoxy, linéaire, ramifié ou cyclisé, renfermant de 7 à 12 atomes de carbone, $R_2$ représente un atome d'hydrogène, un radical alcoyle, linéaire ou ramifié, renfermant de 1 à 6 atomes de carbone, un radical aryle renfermant de 6 à 12 atomes de carbone, un radical aryle renfermant de 6 à 12 atomes de carbone substitué par un ou plusieurs radicaux alcoyles ou alcoxy renfermant de 1 à 6 atomes de carbone, par un ou plusieurs atomes d'halogène ou par un ou plusieurs radicaux nitro ou $R_2$ représente un radical 2-thiényle, un radical alcoxycarbonyle renfermant de 2 à 7 atomes de carbone ou un radical cycloalcoyle renfermant de 3 à 6 atomes de carbone, caractérisé en ce que l'on chauffe un produit de formule (II) :

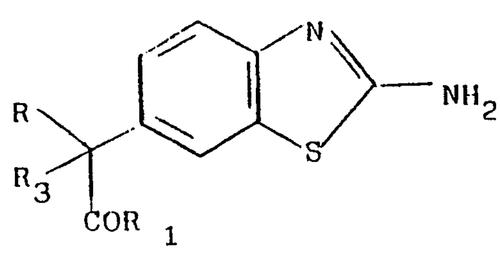

(II)

dans laquelle R, $R_1$ et $R_3$ ont la signification indiquée ci-dessus, avec un produit de formule (III) :

$$R_2-C{\equiv}C-CO_2R_4 \qquad (III)$$

dans laquelle $R_2$ a la signification déjà indiquée et $R_4$ représente un radical alcoyle renfermant de 1 à 3 atomes de carbone, pour obtenir le produit de formule (I) correspondant, que soit l'on isole et si désiré salifie, soit l'on fait réagir ce dernier avec un alcool de formule (IV) :

$$H-R'_1 \qquad (IV)$$

dans laquelle $R'_1$ représente un radical alcoxy linéaire, ramifié ou cyclisé renfermant de 7 à 12 atomes de carbone pour obtenir un composé de formule (I) correspondant dans laquelle $R_1$ a la signification de $R'_1$

2. Procédé selon la revendication 1, caractérisé en ce que l'on fait réagir un produit de formule (V):

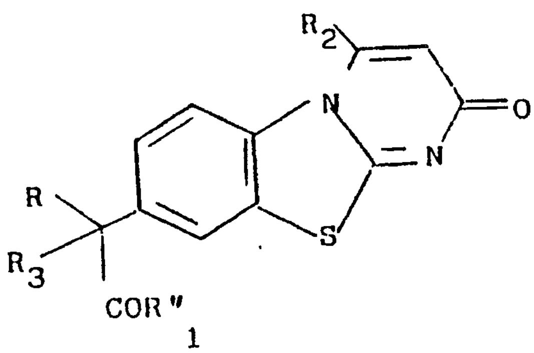

(V)

dans laquelle R, $R_2$ et $R_3$ ont la signification déjà indiquée et $R''_1$ représente un radical hydroxy ou alcoxy renfermant de 1 à 6 atomes de carbone, avec un alcool de formule (VI):

$$H-R_1 \qquad (VI)$$

dans laquelle $R_1$ a la signification déjà indiquée, pour obtenir un produit de formule (I) correspondant que l'on isole et si désiré salifie.

3. Procédé selon la revendication 1, caractérisé en ce que
la réaction du produit de formule (II) avec le propiolate de formule (III) peut être effectuée, au reflux du mélange réactionnel, au sein d'un alcanol et en présence d'un catalyseur.

4. Procédé selon la revendication 3, caractérisé en ce que la réaction du produit de formule (II) avec le produit de formule (III) est effectuée au sein de l'éthanol en présence de palladium.

5. Procédé de préparation tel que défini à la revendication 1, caractérisé en ce que la réaction du produit de formule (II) avec le produit de formule (III) est effectuée à une température de 100 à 180 °C, en l'absence de solvant.

6. Procédé selon la revendication 1 ou 2, caractérisé en ce que l'on utilise au départ des composés de formule (II) ou (V) dans laquelle R et $R_3$ représentent un atome d'hydrogène ou un radical méthyle ou éthyle.

7. Procédé selon la revendication 1 ou 2, caractérisé en ce que l'on utilise au départ des composés dans lesquels R et $R_3$ représentent un atome d'hydrogène ou un radical méthyle ou éthyle, $R_1$ a la signification déjà indiquée, $R_2$ représente un atome d'hydrogène, un radical alcoyle renfermant de 1 à 6 atomes de carbone, un radical phényle, un radical phényle substitué par un ou plusieurs radicaux

méthyle par un ou plusieurs atomes d'halogène ou par un ou plusieurs radicaux nitro ou méthoxy ou $R_2$ représente un radical méthoxy carbonyle.

8. Procédé selon la revendication 1 ou 2, caractérisé en ce que l'on utilise au départ des composés dans lesquels R et $R_3$ representent un atome d'hydrogène ou un radical méthyle ou éthyle, $R_2$ représente un radical phényle et $R_1$ représente un radical 1-méthylcyclohexyloxy ou un radical 1-adamantyloxy.

## Claims

1. New derivatives of pyrimido-[2,1-b]-benzothiazole as well as their addition salts with acids, characterized in that they correspond to general formula (I):

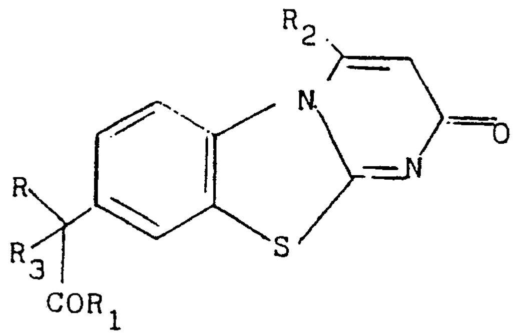

(I)

in which R and $R_3$, identical or different, represent a hydrogen atom, a linear or branched alkyl radical containing 1 to 6 carbon atoms or R and $R_3$ form with the carbon atom to which they are linked, a cycloalkyl radical containing 3 to 6 carbon atoms, $R_1$ represents a linear, branched or cyclized alkoxy radical containing 7 to 12 carbon atoms,
$R_2$ represents a hydrogen atom, a linear or branched alkyl radical containing 1 to 6 carbon atoms, an aryl radical containing 6 to 12 carbon atoms, an aryl radical containing 6 to 12 carbon atoms substituted by one or more alkyl or alkoxy radicals containing 1 to 6 carbon atoms, by one or more halogen atoms or by one or more nitro radicals, or $R_2$ represents a 2-thienyl radical, an alkoxy carbonyl radical containing 2 to 7 carbon atoms or a cycloalkyl radical containing 3 to 6 carbon atoms.

2. Derivatives corresponding to general formula (I) of claim 1, as well as their addition salts with acids, characterized in that in said formula (I), R and $R_3$ represent a hydrogen atom or a methyl or ethyl radical, $R_1$ and $R_2$ have the meaning already indicated.

3. Derivatives corresponding to general formula (I) of claim 1, as well as their addition salts with acids, characterized in that in said formula (I), R and $R_3$ represent a hydrogen atom or a methyl or ethyl radical, $R_1$ has the meaning already indicated, $R_2$ represents a hydrogen atom, an alkyl radical containing 1 to 6 carbon atoms, a phenyl radical, a phenyl radical substituted by one or more methyl radicals, by one or more halogen atoms, or by one or more nitro or methoxy radicals or $R_2$ represents a methoxy carbonyl radical.

4. Derivatives corresponding to formula (I) of claim 1, as well as their salts, characterized in that in said formula (I), R and $R_3$ represent a hydrogen atom or a methyl or ethyl radical, $R_2$ represents a phenyl radical and $R_1$ represents a 1-methyl-cyclohexyloxy radical or a 1-adamantyloxy radical.

5. Any one of the products of formula (I) as defined in claim 1, the names of which follow:
   - alpha-methyl-2-oxo-4-phenyl2Hpyrimido[2,1-b]-benzothiazole-8-acetate of 1-methylcyclohexyl,
   - alpha-methyl-2-oxo-4phenyl2Hpyrimido-[2,1-b]-benzothiazole-8-acetate of 1-adamantyl, as well as their addition salts with acids.

6. Preparation process for new derivatives of pyrimido-[2,1-b]-benzothiazole as well as their addition salts with acids, characterized in that a product of formula (II):

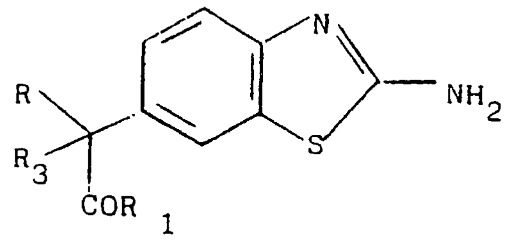

(II)

in which R, $R_1$ and $R_3$ have the meaning indicated in claim 1, is heated with a product of formula (III):

$$R_2 - C{\equiv}C - CO_2R_4 \qquad (III)$$

in which $R_2$ has the meaning already indicated and $R_4$ represents an alkyl radical containing 1 to 3 carbon atoms, in order to obtain the corresponding product of formula (I), which is either isolated and if desired salified, or the latter is reacted with an alcohol of formula (IV):

$$H{-}R'_1 \qquad (IV)$$

in which $R'_1$ represents a linear, branched or cyclized alkoxy radical containing 7 to 12 carbon atoms, in order to obtain a corresponding compound of formula (I) in which $R_1$ has the meaning of $R'_1$, which is isolated and if desired salified.

7. Preparation process for new derivatives of pyrimido-[2,1-b]-benzothiazole as well as their addition salts with acids, characterized in that a product of formula (V):

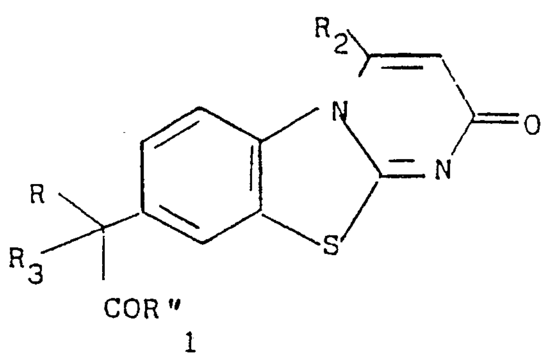

(V)

in which R, $R_2$ and $R_3$ have the meaning already indicated and $R''_1$ represents a hydroxy or alkoxy radical containing 1 to 6 carbon atoms, is reacted with an alcohol of formula (VI):

$$H{-}R_1 \qquad (VI)$$

in which $R_1$ has the meaning already indicated, in order to obtain a corresponding product of formula (I) which is isolated and if desired salified.

8. Preparation process for new derivatives of pyrimido-[2,1-b]-benzothiazole as defined in claim 6, characterized in that the reaction of the product of formula (II) with the product of formula (III) is carried out under reflux of the reaction mixture, in an alkanol and in the presence of a catalyst.

9. Process according to claim 8, characterized in that the reaction of product (II) with the product of

formula (III) is carried out in ethanol in the presence of palladium.

10. Preparation process for new derivatives of pyrimido-[2,l-b]-benzothiazole as defined in claim 6, characterized in that the reaction of the product of formula (II) with the product of formula (III) is carried out, at a temperature of 100 to 180 °C, in the absence of a solvent.

11. Medicaments, characterized in that they are constituted by the new derivatives of pyrimido-[2,l-b]-benzothiazole as defined by formula (I) of claim 1 as well as by their addition salts with pharmaceutically acceptable acids.

12. Medicaments, characterized in that they are constituted by the new derivatives as defined in any one of claims 2 to 5, as well as by their addition salts with pharmaceutically acceptable acids.

13. Pharmaceutical compositions, characterized in that they contain as active ingredient, at least one of the medicaments as defined in any one of claims 11 or 12.

Claims for the following Contracting State : AT

1. Preparation process for new derivatives of pyrimido-[2,1-b]-benzothiazole as well as their addition salts with acids, corresponding to general formula (I):

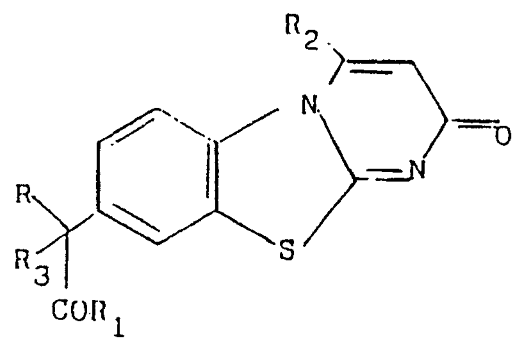

(I)

in which R and $R_3$, identical or different, represent a hydrogen atom, a linear or branched alkyl radical containing 1 to 6 carbon atoms or R and $R_3$ form with the carbon atom to which they are linked, a cycloalkyl radical containing 3 to 6 carbon atoms, $R_1$ represents a linear, branched or cyclized alkoxy radical containing 7 to 12 carbon atoms, $R_2$ represents a hydrogen atom, a linear or branched alkyl radical containing 1 to 6 carbon atoms, an aryl radical containing 6 to 12 carbon atoms, an aryl radical containing 6 to 12 carbon atoms substituted by one or more alkyl or alkoxy radicals containing 1 to 6 carbon atoms, by one or more halogen atoms or by one or more nitro radicals, or $R_2$ represents a 2-thienyl radical, an alkoxycarbonyl radical containing 2 to 7 carbon atoms or a cycloalkyl radical containing 3 to 6 carbon atoms, characterized in that a product of formula (II):

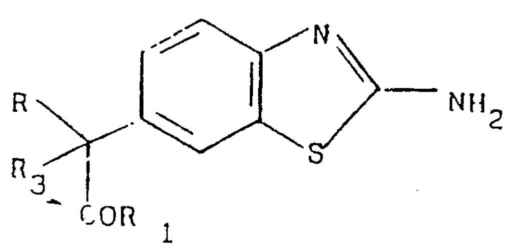

(II)

in which R, $R_1$ and $R_3$ have the meaning indicated above, is heated with a product of formula (III):

$$R_2 - C \equiv C - CO_2R_4 \qquad (III)$$

in which $R_2$ has the meaning already indicated and $R_4$ represents an alkyl radical containing 1 to 3 carbon atoms, in order to obtain the corresponding product of formula (I), which is either isolated and if desired salified, or the latter is reacted with an alcohol of formula (IV):

$$H-R'_1 \qquad (IV)$$

in which $R'_1$ represents a linear, branched or cyclized alkoxy radical containing 7 to 12 carbon atoms, in order to obtain a corresponding compound of formula (I) in which $R_1$ has the meaning of $R'_1$.

2. Process according to claim 1, characterized in that a product of formula (V):

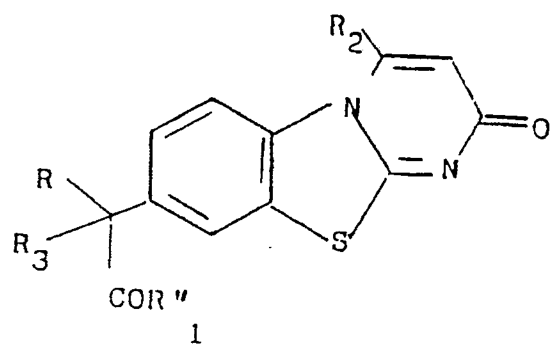

(V)

in which R, $R_2$ and $R_3$ have the meaning already indicated and $R''_1$ represents a hydroxy or alkoxy radical containing 1 to 6 carbon atoms, is reacted with an alcohol of formula (VI):

$$H-R_1 \qquad (VI)$$

in which $R_1$ has the meaning already indicated, in order to obtain a corresponding product of formula (I) which is isolated and if desired salified.

3. Process according to claim 1, characterized in that the reaction of the product of formula (II) with the propiolate of formula (III) is carried out under reflux of the reaction mixture, in an alkanol and in the presence of a catalyst.

4. Process according to claim 3, characterized in that the reaction of the product of formula (II) with the product of formula (III) is carried out in ethanol in the presence of palladium.

5. Preparation process as defined in claim 1, characterized in that the reaction of the product of formula (II) with the product of formula (III) is carried out, at a temperature of 100 to 180 °C, in the absence of a solvent.

6. Process according to claim 1 or 2, characterized in that at the start the compounds of formula (II) or (V) are used, in which R and $R_3$ represent a hydrogen atom or a methyl or ethyl radical.

7. Process according to claim 1 or 2, characterized in that at the start the compounds are used in which R and $R_3$ represent a hydrogen atom or a methyl or ethyl radical, $R_1$ has the meaning already indicated, $R_2$ represents a hydrogen atom, an alkyl radical containing 1 to 6 carbon atoms, a phenyl radical, a phenyl radical substituted by one or more methyl radicals, by one or more halogen atoms, or by one or more nitro or methoxy radicals or $R_2$ represents a methoxy carbonyl radical.

8. Process according to claim 1 or 2, characterized in that at the start the compounds are used in which R and $R_3$ represent a hydrogen atom or a methyl or ethyl radical, $R_2$ represents a phenyl radical and $R_1$ represents a 1-methyl-cyclohexyloxy radical or a 1-adamantyloxy radical.

## Ansprüche

1. Neue Derivate des Pyrimido[2,1-b]benzothiazols sowie ihre Additionssalze mit Säuren, dadurch gekennzeichnet, daß sie der allgemeinen Formel (I)

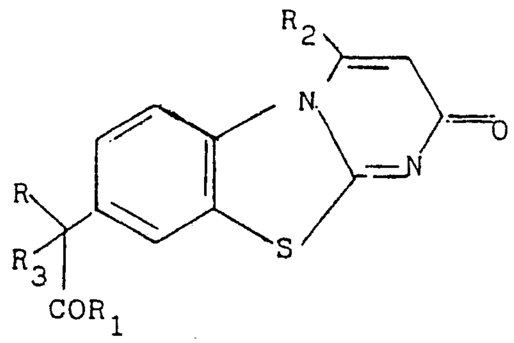

(I)

entsprechen, worin R und $R_3$, die identisch oder verschieden sind, ein Wasserstoffatom, einen geraden oder verzweigten Alkrest mit 1 bis 6 Kohlenstoffatomen bedeuten oder R und $R_3$ bilden mit dem Kohlenstoffatom, an das sie gebunden sind, einen Cycloalkylrest mit 3 bis 6 Kohlenstoffatomen, $R_1$ bedeutet einen geraden oder verzweigten oder cyclisierten Alkoxyrest mit 7 bis 12 Kohlenstoffatomen, $R_2$ bedeutet ein Wasserstoffatom, einen geraden oder verzweigten Alkylrest mit 1 bis 6 Kohlenstoffatomen, einen Arylrest mit 6 bis 12 Kohlenstoffatomen, einen Arylrest mit 6 bis 12 Kohlenstoffatomen, substituiert durch einen oder mehrere Alkyl- oder Alkoxyreste mit 1 bis 6 Kohlenstoffatomen, durch ein oder mehrere Halogenatome oder durch einen oder mehrere Nitroreste, oder $R_2$ bedeutet einen 2-Thienylrest, einen Alkoxycarbonylrest mit 2 bis 7 Kohlenstoffatomen oder einen Cycloalkylrest mit 3 bis 6 Kohlenstoffatomen.

2. Derivate der allgemeinen Formel (I) gemäß Anspruch 1 sowie ihre Additionssalze mit Säuren, dadurch gekennzeichnet, daß in der Formel (I) R und $R_3$ ein Wasserstoffatom oder einen Methyl- oder Ethylrest bedeuten, $R_1$ und $R_2$ die angegebene Bedeutung haben.

3. Derivate der allgemeinen Formel (I) gemäß Anspruch 1 sowie ihre Additionssalze mit Säuren, dadurch gekennzeichnet, daß in der Formel (I) R und $R_3$ ein Wasserstoffatom oder einen Methyl- oder Ethylrest bedeuten, $R_1$ die bereits angegebene Bedeutung hat, $R_2$ ein Wasserstoffatom, einen Alkylrest mit 1 bis 6 Kohlenstoffatomen, einen Phenylrest, einen Phenylrest, substituiert durch einen oder mehrere Methylreste, durch ein oder mehrere Halogenatome oder durch einen oder mehrere Nitro- oder Methoxtreste, bedeutet oder $R_2$ einen Methoxycarbonylrest darstellt.

4. Derivate der Formel (I) gemäß Anspruch 1 sowie ihre Salze, dadurch gekennzeichnet, daß in der Formel (I) R und $R_3$ ein Wasserstoflatom oder eine Methyl- oder Ethylgruppe bedeuten, $R_2$ einen Phenylrest wiedergibt und $R_1$ einen 1-Methylcyclohexyloxy- oder 1-Adamantyloxyrest darstellt.

5. Eines der Produkte der Formel (I), wie in Anspruch 1 definiert, mit folgendem Namen:
α-Methyl-2-oxo-4-phenyl-2H-pyrimido [2,1-b]benzothiazol-8-essigsäure-1-methylcyclohexylester,
α-Methyl-2-oxo-4-phenyl-2H-pyrimido [2,1-b]benzothiazol-8-essigsäure-1-adamantylester sowie ihre Additionssalze mit Säuren.

6. Verfahren zur Herstellung der neuen Derivate des Pyrimido[2,1-b]benzothiazols sowie ihrer Additionssalze mit Säuren, dadurch gekennzeichnet, daß man ein Produkt der Formel (II)

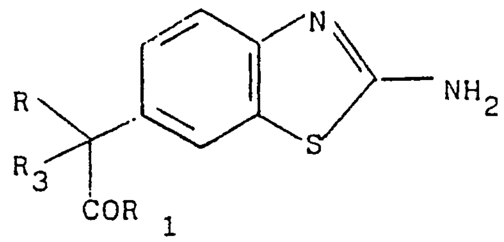

(II)

worin R, $R_1$ und $R_3$ die in Anspruch 1 angegebene Bedeutung haben, mit einem Produkt der Formel (III)

$$R_2 - C \equiv C - CO_2R_4 \qquad (III)$$

worin $R_2$ die angegebene Bedeutung hat und $R_4$ einen Alkylrest mit 1 bis 3 Kohlenstoffatomen bedeutet, erhitzt, um das entsprechende Produkt der Formel (I) zu erhalten, und es entweder isoliert und gewünschtenfalls in ein Salz überfuhrt oder das letztere mit einem Alkohol der Formel (IV)

$$H - R'_1 \qquad (IV)$$

zur Reaktion bringt, worin $R'_1$ einen geraden, verzweigten oder cyclisierten Alkoxyrest mit 7 bis 12 Kohlenstoffatomen darstellt, um eine entsprechende Verbindung der Formel (I) zu erhalten, worin $R_1$ die Bedeutung von $R'_1$ hat, das man isoliert und gewünschtenfalls in ein Salz Überführt.

7. Verfahren zur Herstellung der neuen Derivate des Pyrimido [2,1-b]benzothiazols sowie ihrer Additionssalze mit Säuren, dadurch gekennzeichnet, daß man ein Produkt der Formel (v)

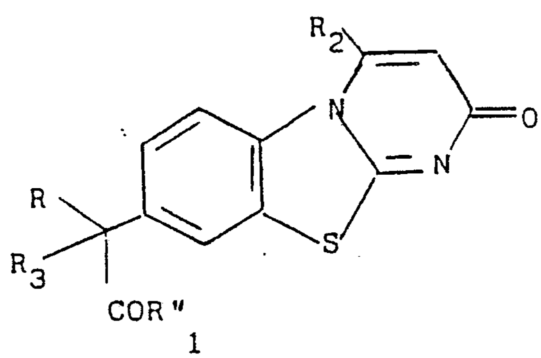

(V)

worin R, R2 und $R_3$ die angegebene Bedeutung haben und $R''_1$ einen Hydroxy- oder Alkoxyrest mit 1 bis 6 Kohlenstoffatomen darstellt, mit einem Alkohol der Formel (VI)

$$H - R_1 \qquad (VI)$$

worin $R_1$ die angegebene Bedeutung hat, zur Reaktion bringt, um ein entsprechendes Produkt der Formel (I) zu erhalten, das man isoliert und gewünschtenfalls in ein Salz überführt.

8. Verfahren zur Herstellung von neuen Derivaten des Pyrimido[2,1-b]benzothiazols, wie in Anspruch 6 definiert, dadurch gekennzeichnet, daß die Reaktion des Produkts der Formel (II) mit dem Produkt der Formel (III) unter Rückfluß des Reaktionsgemisches durchgeführt wird in einem Alkanol und in Gegenwart eines Katalysators.

9. Verfahren gemäß Anspruch 8, dadurch gekennzeichnet, daß die Reaktion des Produkts (II) mit dem Produkt der Formel (III) in Ethanol in Gegenwart von Palladium durchgeführt wird.

10. Verfahren zur Herstellung der neuen Derivate des Pyrimido[2,1-b]benzothiazols, wie in Anspruch 6 definiert, dadurch gekennzeichnet, daß die Reaktion des Produkts der Formel (II) mit dem Produkt der Formel (III) bei einer Temperatur von 100 bis 180 °C in Abwesenheit von Lösungsmittel durchgeführt wird.

11. Arzneimittel, dadurch gekennzeichnet, daß sie aus den neuen Derivaten des Pyrimido[2,1-b]-benzothiazols, wie in Formel (I) von Anspruch 1 definiert, sowie ihren Additionssalzen mit pharmazeutisch annehmbaren Säuren bestehen.

12. Arzneimittel, dadurch gekennzeichnet, daß sie aus den neuen Derivaten, wie sie in einem der Ansprüche 2 bis 5 definiert sind, sowie ihren Additionssalzen mit pharmazeutisch annehmbaren Säuren bestehen.

13. Pharmazeutische Zusammensetzungen, dadurch gekennzeichnet, daß sie als aktives Prinzip mindestens eines der Arzneimittel, wie sie in einem der Ansprüche 11 oder 12 definiert sind, enthalten.

Patentansprüche für folgenden Vertragsstaat : AT

1. Verfahren zur Herstellung von neuen Derivaten des Pyrimido[2,1-b]benzothiazols sowie ihrer Additionssalze mit Säuren der allgemeinen Formel (I)

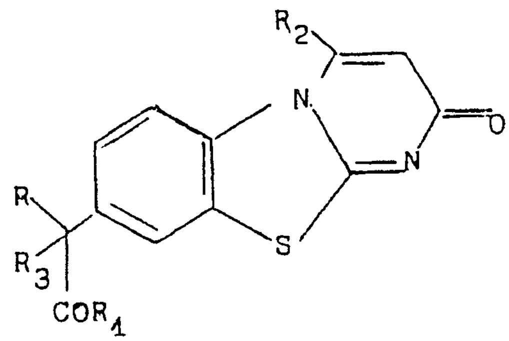

(I)

worin R und $R_3$, die identisch oder verschieden sind, ein Wasserstoffatom, einen geraden oder verzweigten Alkylrest mit 1 bis 6 Kohlenstoffatomen bedeuten oder R und $R_3$ mit dem Kohlenstoffatom, an das sie gebunden sind, einen Cycloalkylrest mit 3 bis 6 Kohlenstoffatomen bilden, $R_1$ einen geraden, verzweigten oder cyclisierten Alkoxyrest mit 7 bis 12 Kohlenstoffatomen darstellt, $R_2$ ein Wasserstoffatom, einen geraden oder verzweigten Alkylrest mit 1 bis 6 Kohlenstoffatomen, einen Arylrest mit 6 bis 12 Kohlenstoffatomen, einen Arylrest mit 6 bis 12 Kohlenstoffatomen, substituiert durch einen oder mehrere Alkyl- oder Alkoxyreste mit 1 bis 6 Kohlenstoffatomen, durch ein oder mehrere Halogenatome oder durch einen oder mehrere Nitroreste, bedeutet oder $R_2$ einen 2-Thienylrest, einen Alkoxycarbonylrest mit 2 bis 7 Kohlenstoffatomen oder einen Cycloalkylrest mit 3 bis 6 Kohlenstoffatomen darstellt, dadurch **gekennzeichnet,** daß man ein Produkt der Formel (II)

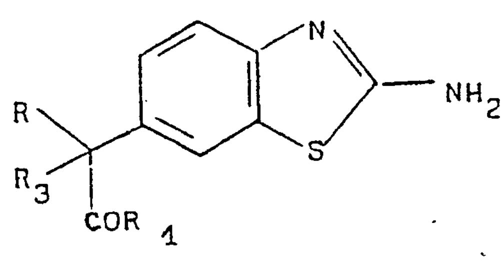

(II)

worin R, $R_1$ und $R_3$ die oben angegebene Bedeutung haben, mit einem Produkt der Formel (III)

$$R_2 - C \equiv C - CO_2R_4 \qquad (III)$$

wobei $R_2$ die bereits angegebene Bedeutung hat und $R_4$ einen Alkylrest mit 1 bis 3 Kohlenstoffatomen darstellt, erhitzt, um das entsprechende Produkt der Formel (I) zu erhalten, das man entweder isoliert und gewünschtenfalls in ein Salz überführt oder letzteres mit einem Alkohol der Formel (IV)

$$H - R'_1 \qquad (IV)$$

worin $R'_1$ einen geraden, verzweigten oder cyclisierten Alkoxyrest mit 7 bis 12 Kohlenstoffatomen darstellt, zur Reaktion bringt, um eine entsprechende Verbindung der Formel (I) zu erhalten, worin $R_1$ die Bedeutung von $R'_1$ hat.

**2.** Verfahren gemäß Anspruch 1, dadurch gekennzeichnet, daß man ein Produkt der Formel (V)

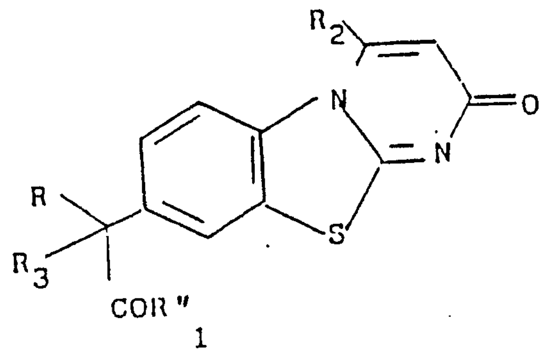

(V)

worin R, $R_2$ und $R_3$ die bereits angegebene Bedeutung haben und $R''_1$ einen Hydroxy- oder Alkoxyrest mit 1 bis 6 Kohlenstoffatomen darstellt, mit einem Alkohol der Formel (VI)

$$H - R_1 \qquad (VI)$$

zur Reaktion bringt, worin R1 die angegebene Bedeutung hat, um ein entsprechendes Produkt der Formel (I) zu erhalten, das man isoliert und gewünschtenfalls in ein Salz überführt.

**3.** Verfahren gemäß Anspruch 1, dadurch gekennzeichnet, daß die Reaktion des Produkts der Formel (II) mit dem Propiolat der Formel (III) unter Rückfluß des Reaktionsgemisches in einem Alkanol und in Gegenwart eines Katalysators durchgeführt werden kann.

**4.** Verfahren gemäß Anspruch 3, dadurch gekennzeichnet, daß die Reaktion des Produkts der Formel (II) mit dem Produkt der Formel (III) in Ethanol in Gegenwart von Palladium durchgeführt wird.

**5.** Herstellungsverfahren, wie in Anspruch 1 definiert, dadurch gekennzeichnet, daß die Reaktion des Produkts der Formel (II) mit dem Produkt der Formel (III) bei einer Temperatur von 100 bis 180 °C in Abwesenheit von Lösungsmittel durchgeführt wird.

**6.** Verfahren gemäß Anspruch 1 oder 2, dadurch gekennzeichnet, daß man zu Beginn Verbindungen der Formel (II) oder (V) verwendet, worin R und $R_3$ ein Wasserstoffatom oder einen Methyl- oder Ethylrest bedeuten.

**7.** Verfahren gemäß Anspruch 1 oder 2, dadurch gekennzeichnet, daß man zu Beginn Verbindungen verwendet, worin R und $R_3$ ein Wasserstoffatom oder einen Methyloder Ethylrest bedeuten, $R_1$ die bereits angegebene Bedeutung hat, $R_2$ ein Wasserstoffatom, einen Alkylrest mit 1 bis 6 Kohlenstoffatomen, einen Phenylrest, einen Phenylrest, substituiert durch einenoder mehrere Methylreste, durch ein oder mehrere Halogenatome oder durch einen oder mehrere Nitro- oder Methoxyreste, bedeutet oder $R_2$ einen Methoxycarbonylrest darstellt.

8. Verfahren gemäß Anspruch 1 oder 2, dadurch gekennzeichnet, daß man als Ausgangsmaterialien Verbindungen verwendet, worin R und $R_3$ ein Wasserstoffatom oder einen Methyl- oder Ethylrest bedeuten, $R_2$ einen Phenylrest darstellt und $R_1$ einen 1-Methylcyclohexyloxy-oder 1-Adamantyloxyrest bedeutet.